# EUROPEAN PATENT SPECIFICATION

(11) **EP 4 080 264 B1**
(45) Date of publication and mention of the grant of the patent: **18.02.2026**
(21) Application number: 21185712.3
(22) Date of filing: 15.07.2021
(51) Int. Cl.: C12M 1/34, G02B 21/34, G02B 21/26

(54) **MICROSCOPE**
MIKROSKOP
MICROSCOPE

(30) Priority: 23.04.2021 EP 21170309
(43) Date of publication of application: 26.10.2022
(73) Proprietor: Leica Microsystems CMS GmbH, 35578 Wetzlar (DE)
(72) Inventor: PELZER, Patric, Dr., 35578 Wetzlar (DE); BENDER, Claus, 35614 Asslar-Werdorf (DE)
(74) Representative: Schaumburg und Partner Patentanwälte mbB

(56) References cited:
- EP-A1- 2 031 429
- WO-A1-2017/079682
- JP-A- 2006 187 206
- US-A- 5 851 790
- US-A1- 2021 071 128

## Description

### Technical field

The present inventive concept generally relates to microscopes for examining a sample, more particularly, for imaging a sample, the microscope comprising a microscope stage for receiving the sample to be examined and further comprising a microscope housing enclosing the microscope stage. Such microscopes can particularly be used for examination of a sample in a sample chamber, which is incubated with an incubation atmosphere adapted to the sample. In general, however, also other uses and applications of such microscopes can be envisaged.

### Background

In the field of microscopic examination of living samples, like cells, it is common practice to make use of incubated sample chambers and/or to supply a reagent to the sample before or during examination of the sample. Such reagents are supplied to a sample for triggering biological or chemical reactions with the sample or just for preserving the sample. Usually, the sample itself is located in multi-well-plates, Petri dishes or microfluidic systems on a microscope table/microscope stage. Pumps may be used for supplying the reagent to the sample, on the other hand, manual pipetting may be carried out.

Additionally or alternatively, the sample is examined in an incubation atmosphere which provides best possible conditions to a living sample regarding temperature, humidity and gas, particularly CO₂, composition. While access to the sample chamber is often needed for supplying a reagent to the sample and/or for replacing the sample, and sometimes also for adjusting optical components of the microscope in the sample chamber, escape of incubation atmosphere should be limited.

Furthermore, in general, the sample handling, particularly exchanging of samples, manipulating samples, supplying reagent to the sample etc., should be facilitated.

US 2006/0092506 A1 discloses a microscope including a high humidity space that is maintained at a predetermined gas temperature for culture of a living sample. An embodiment of this microscope has three spaces, namely a cell culture space of a controlled incubation atmosphere where temperature, humidity and CO₂₋concentration are controlled, an observation space including the objective lens of the microscope, the observation space being only temperature controlled, and an outside space provided with an imaging lens and a digital camera, on the one hand, and a light source for transmissive lighting, one the other hand. The cell culture space is covered with a cover box, which serves as an opening/closing unit and is provided with a thermal insulation at its inside. The cover box is provided with a hinge so that the cover box can be opened/closed with rotation around the hinge. Thus, the sample can be taken in and out from the cell culture space by opening the hinged cover box. When the cover box is opened, the cell culture space communicates with the outside space.

Document US 2021/0071128 A1 relates to a microscope apparatus having two separate doors, for working access and maintenance access. Documents EP 2 031 429 A1, JP 2006-187206 A and WO 2017/079682 A1 also relate to microscope apparatuses. Further reference is made to US 5 851 790 A, which discloses a cytogenetic chamber having a laterally-swinging access door with hand-insertion ports.

### Summary

In view of the above tasks in modern microscopy, especially for examining living samples, there is a need for an improved microscope.

The invention is defined by claim 1. The present invention provides a microscope for examining a sample, the microscope comprising a microscope stage for receiving the sample to be examined and further comprising a microscope housing enclosing the microscope stage, wherein the microscope housing comprises a first access door and a second access door, whereby both, the first access door and the second access door, upon opening provide different ways of access to the microscope stage, or, in other words, different ways of direct access into the interior of the microscope housing. In principle, the first and second access doors can be arranged at the same side of the microscope housing, particularly at its front side, or at opposite sides of the microscope housing, particularly at opposite sidewalls of the microscope housing, or one access door at the front side, the other one at a sidewall of the microscope housing. Further, in principle, the first and the second access doors can be of the same size or of different sizes as long as the first and the second access doors, in their opened state, provide different ways of access to the microscope stage.

The term "different ways of access to the microscope stage" includes the possible ways to reach the microscope stage through respective openings provided by the first and second opened access doors. "Different ways of access" thus require different sizes of the respective openings and/or different locations of the respective openings provided by the opened access doors. One or more different access door parameters (like at least location and size) result in different spherical angles having their base point on the microscope stage and enveloping the possible ways of access to the microscope stage through an opened access door. A user of the microscope can thus decide which access door to use according to the current demands of accessibility. These demands typically depend on whether the user needs direct access to the sample, direct access to other areas next to the microscope stage or on the microscope stage, e.g. for placing an accessory device like a pump or a reagent container next to the microscope stage, or pipetting a sample or exchanging a sample or adjusting other microscope components in the sample chamber around the microscope stage, e.g. an illumination unit or parts of an incubation system. For example, opening a sidewall door may have a lower impact on the incubation atmosphere than opening a front side door of the microscope housing. In another example, setting up an accessory device that should be placed next to the microscope stage may require access through a larger door than exchanging a sample after incubation atmosphere equilibration has been reached.

In an embodiment of the invention, the first access door is configured by a first housing part of the microscope housing and/or the second access door is configured by a second housing part of the microscope housing. Thus, an access door may be hinged housing part, but an access door may also be a slidable door in an opening of the housing. It should be noted, however, that any access door (in the meaning of the present invention), at least in its closed state, can be regarded as a housing part as it contributes to the "housing" and, in its opened state, provides direct access into the interior of the microscope housing.

In an embodiment of the invention, the first housing part is larger than the second housing part and/or the opening provided by opening the first access door is larger than the opening provided by opening the second access door. Thus, these openings can be dimensioned in a way to provide easy access to the microscope stage and its surroundings through the first access door, and, especially in case of an incubated sample chamber, to provide a more direct access to the sample itself through the second access door while minimizing disturbances of the sample inside the sample chamber, minimizing light contamination from ambient light and/or minimizing disturbances and an escape of incubation atmosphere in the sample chamber.

For ease of illustration but without loss of generality, the following embodiments of a microscope according to the present invention are based on a microscope configuration where the microscope stage is enclosed by the microscope housing to form a separate sample chamber. This sample chamber may be incubated. Other microscope components like an illumination unit may also be arranged inside the sample chamber. Such a microscope configuration may further comprise another space separated from the sample chamber and accommodating other microscope components like imaging optics. Such a configuration is typically used for transmitted-light microscopes typically used for the examination of living samples. As noted above, other configurations of a microscope according to the present invention are possible and are not meant to be excluded. For instance, the illumination unit of the microscope may be placed outside of the sample chamber and/or an incident-light microscope can be used.

Further, it is noted that the first and the second access doors according to the present invention need not to be arranged separated from each other but rather one access door is part of the other access door. Especially in the embodiment where the first housing part or the first access door is larger than the second housing part or the second access door, the second access door is included in the first access door. Such a configuration may be called "door-in-door"-configuration.

It is expedient to configure the second access door as a hinged door or as a sliding door. While a sliding door can be configured to consume very little space when opened, a hinged door may be preferably configured such that the door is pivoted down in the opened state to form a horizontal or essentially horizontal work surface. Such a work surface can provide a storage area, a table or a shelf, and can thus be used for putting down things like reagent containers, laboratory bottles, pipettes, sample containers like multi-well-plates, Petri dishes and the like. Apart from its function to provide access to the microscope stage, the hinged door can thus have a second function of providing a work surface.

According to claim 1 of the present invention, the microscope comprises a third door which, in its closed state, covers the second access door when in its closed state. Generally, but particularly also in this embodiment, it is advantageous if the second access door is formed in a front panel of the housing. The second and the third doors can form a "double-wall"-configuration wherein the third door needs to be opened in order to gain access to the second access door, which, in its opened state, provides access to the microscope stage. In a preferred embodiment, the second access door is, at least in part, transparent such that a user is able to visually control the status of the sample on the microscope stage without the need to open the second access door. In this context, it should be noted that it may be generally of advantage to provide at least one of the access doors with an inspection window and/or to use a transparent material enabling a user to look through the transparent access door.

In the above embodiment of a microscope comprising a third door it is particularly advantageous to form the second access door as a transparent sliding door and to form the third door as an opaque door, which is particularly larger than the second access door and which is preferably a hinged door. Having an opaque third door protects the sample from light contamination from ambient light as soon as the third door is in its closed state when having a transparent second access door.

It is further advantageous if the third door, in its closed state, is embodied as at least a part of a front cover of the microscope housing, the second access door serving for opening and closing the corresponding opening in the microscope housing. Generally, the second access door is preferably formed in a first panel of the microscope housing. In the embodiment of a microscope comprising the third door, it is preferred if the third door covers the part of the front panel including the second access door when the third door is in its closed state. Preferably, the third door forms at least a part of a front cover such that the microscope forms a closed entity, particularly in the form of a block, when the third door is closed. The "double-wall" configuration will be further described below in connection with the figures.

In another embodiment, which is not covered by the present claims, the microscope comprises a third door which includes the second access door. Instead of having a "double-wall" configuration, this configuration corresponds to a "door-in-door" configuration in respect of the second access door and the third door.

By opening the second access door a corresponding opening is uncovered, which provides access to the microscope stage. Opening the third door uncovers a corresponding opening, which at least corresponds to the opening uncovered by the second access door. The opening uncovered by the third door may, however, be bigger than the one uncovered by the second access door. It may be expedient if the opening uncovered by the third door is included in a front panel of the housing of the microscope. By closing the third door, preferably the front panel including said opening is covered by the third door. In this case, by opening the second access door, while the third door is in its closed state, a part of said opening in the front cover is uncovered. Examples of this embodiment will be described in more detail in connection with the figures below.

In an embodiment of the present invention, the first access door configured by the first housing part comprises a hinge to form a hinged lid (or hinged door or hinged cap or hinged hatch) of the microscope housing. The hinged lid of the microscope housing, in its opened state, provides direct access to the microscope stage by being hinged up or down or sideward. In this embodiment, it is particularly preferred if the hinged lid carries the second access door and, particularly, if a third door is present, also the third door.

By opening the hinged lid, a user opens a preferably bigger part of the microscope housing compared to an opening of the second access door and - if present - compared to an opening of the third door. Typically, the hinged lid is opened if a user needs access to the interior of the microscope housing, particularly to the interior of the sample chamber and its surroundings, for instance, to adjust optical components and/or the microscope stage. Also, this embodiment will be described in further detail below in connection with the figures.

As already discussed above, according to the invention the second access door forms a part of the first access door and it is preferred if the first housing part of the microscope housing includes the second housing part of the microscope housing. This allows a user to reach the microscope stage in two different ways from the same side, particularly the front side, of the microscope.

In an embodiment where a third door of the microscope is present, it is preferred if the third door is coupled to the first access door. In this case, the third door may be mounted to the first access door particularly by a hinge mechanism. One possible implementation of this embodiment is a "door-in-door" configuration where the third door is coupled to the first access door with the further option that the second access door is included in the third door. The third door would then also form an access door as it provides direct access to the microscope stage. Such an embodiment will also be described in further detail below in connection with the figures.

As also already discussed above, it is preferred if the second access door is a hinged door which, in an open state, is configured as a work surface and, in a closed state, forms at least a part of the microscope housing.

In embodiments where the microscope comprises a third door it is preferred if the third door is a hinged door which, in an open state, is configured as a work surface and, in a closed state, forms at least a part of the microscope housing. In such embodiments, especially if the third door covers or includes the second access door, it is preferred if the third door assumes the function of a work surface.

It should be noted that any doors of the microscope of the present inventive concept can either be operated manually or electromechanically, e.g. by remote control or by control of a control unit of the microscope.

In a preferred embodiment, the microscope housing comprises a sample chamber, which sample chamber is bounded at least by the upper side of the microscope stage configured to receive the sample, and is further bounded at least by the first access door and by the second access door, when each of them is in the closed state. If a third door is present which includes the second door, the sample chamber is preferably also bounded at least by the third door in its closed state. In a preferred embodiment, the sample chamber comprises and/or is connected to an incubation module for incubating the sample chamber. These embodiments are further discussed in the embodiments according to the Figures below.

In another preferred embodiment, the sample chamber comprises an illumination unit for generating an illumination beam for illuminating the sample when received in the microscope stage. This embodiment of an transmitted-light microscope will be further discussed below.

As used herein the term "and/or" includes any and all combinations of one or more of the associated listed items and may be abbreviated as "/".

Although some aspects have been described in the context of an apparatus, it is clear that these aspects also represent a description of a corresponding method, where a block or device corresponds to a method step or a feature of a method step. Analogously, aspects described in the context of a method step also represent a description of a corresponding block or item or feature of a corresponding apparatus.

Short description of the figures
- Figure 1: schematically shows an embodiment of the present invention where the first access door is in its opened state,
- Figure 2: schematically shows an embodiment of the present invention where the first access door is closed,
- Figure 3: schematically shows the embodiment of Figure 2 where the second access door is opened,
- Figure 4: schematically shows an embodiment similar to that of Figure 2,
- Figure 5: schematically shows an embodiment where all doors of the microscope are closed,
- Figure 6: schematically shows an embodiment where the first access door is closed while the second access door is opened,
- Figure 7: schematically shows another embodiment of a microscope where all the doors are closed,
- Figure 8: schematically shows the embodiment of Figure 7 in an opened state,
- Figure 9: schematically shows an embodiment similar to that of Figure 7 showing a microscope with closed doors and including an incubation module adapted at its backside,
- Figure 10: schematically shows the embodiment of Figure 9 with an opened first access door, and
- Figure 11: schematically shows the embodiment of Figure 9 with an opened second access door.

The Figures are discussed comprehensively, same reference signs designating same or at least structurally identical components.

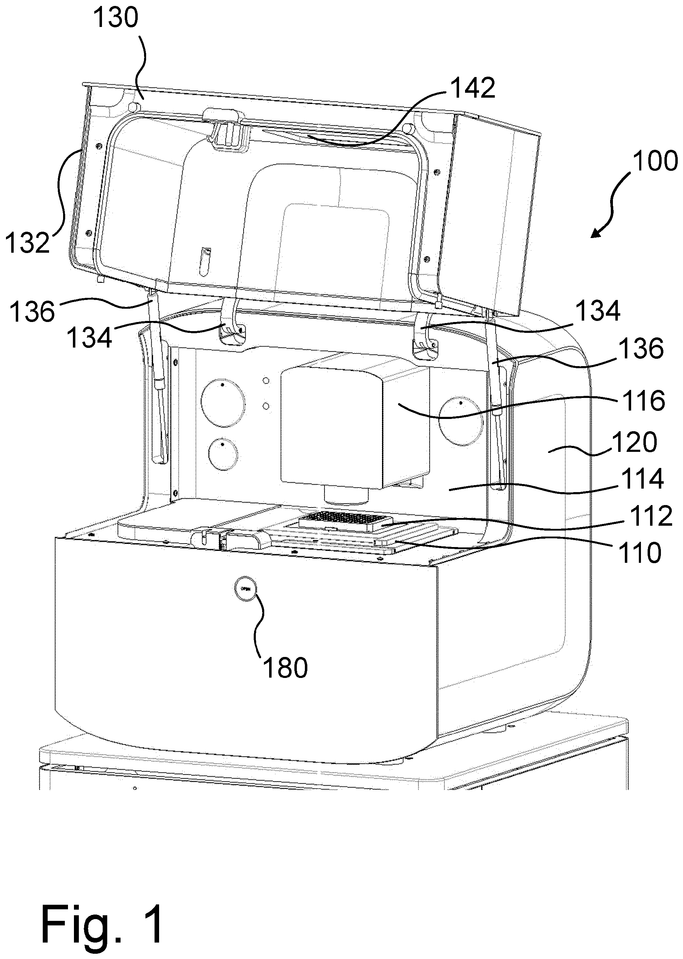

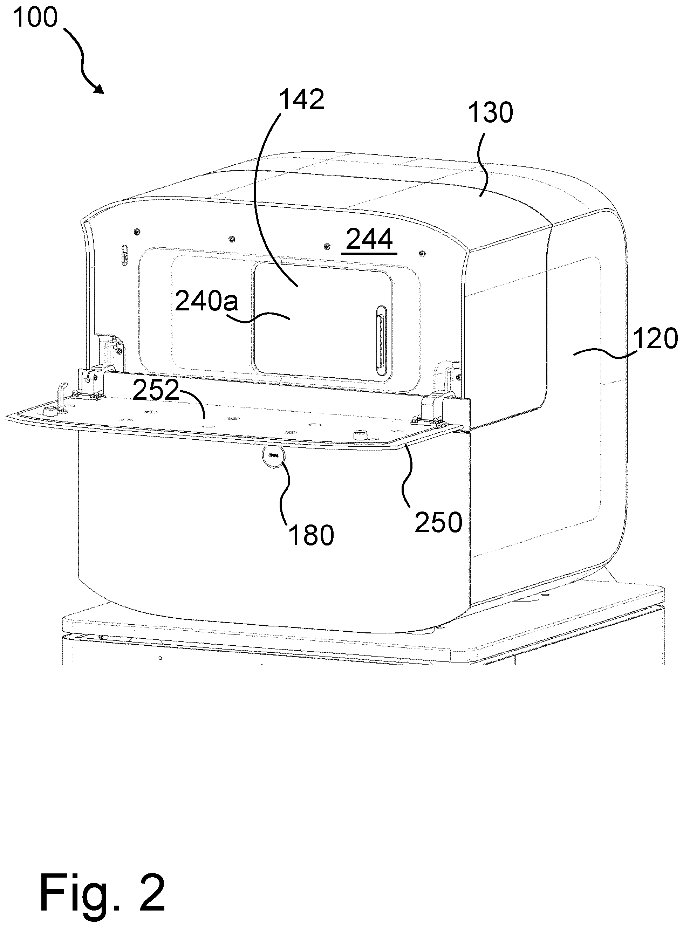

Figure 1 schematically shows an embodiment of a microscope of the present inventive concept, the microscope being designated 100. The microscope 100 comprises a microscope stage 110 for receiving a sample 112 to be examined/imaged and further comprises a microscope housing 120 enclosing the microscope stage 110. In this embodiment, the sample 112 is included in a multi-well-plate as shown in Figure 1. The microscope 100 further comprises a first access door 130 configured by a first housing part 132 of the microscope housing 120. In the embodiment shown in Figure 1, this first access door 130 is in an open state, the corresponding opening providing full access to the interior of a sample chamber 114 including the microscope stage 110. The microscope 100 further comprises a second access door generally depicted 240 (see reference sign 240a in Figure 2) configured by a second housing part 142 of the microscope housing 120, this second housing part being formed by the closed second access door. In this embodiment, the first access door 130 includes the second access door 240a (see Figure 2).

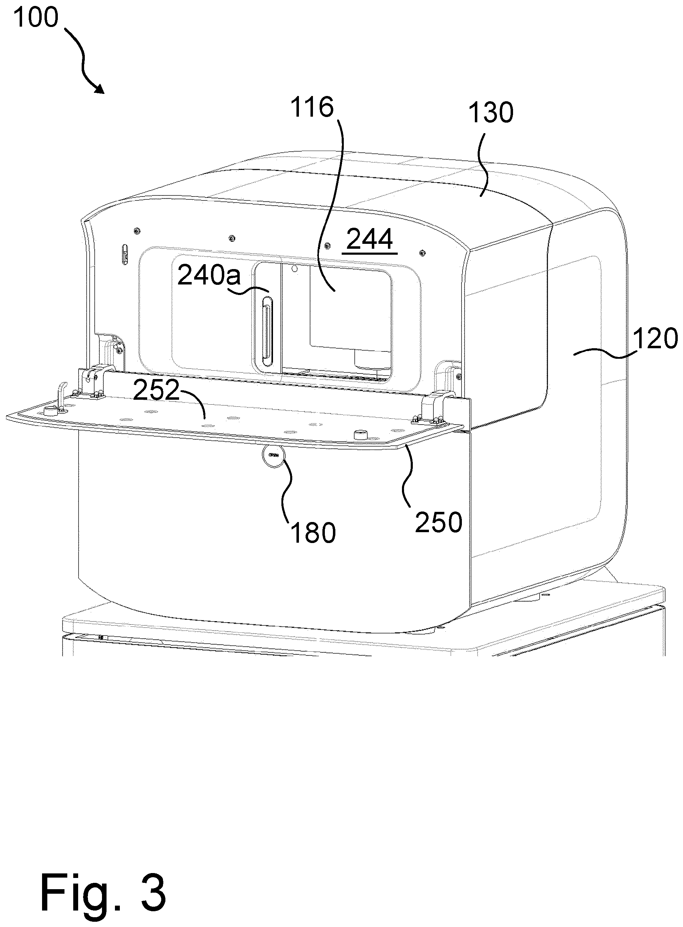

As shown in Figure 2, the second access door 240 is a sliding door 240a, which can be opened by a sliding movement as shown in Figure 3. As can be best seen from Figures 1 and 3, the first access door 130 and the second access door 240a, upon their opening, provide different ways of access to the microscope stage 110. The different sizes and the different locations of the first and second access doors result in different spherical angles enveloping possible ways of access to the microscope stage through the respective opened access doors. While the opened first access door 130 provides access to the microscope stage 110 through a wide variety of directions, the opened second access door 240a only provides a by far restricted access to the microscope stage 110.

The embodiment according to claim 1 shown in Figures 1 to 3 represent a "door-in-door" configuration as the second access door 240a is part of the first access door 130. As can be seen from Figure 1, the first access door 130 configured by the first housing part 132 comprises hinges 134 to form a hinged lid of the microscope housing 120, opening and closing of which being damped by gas springs 136.

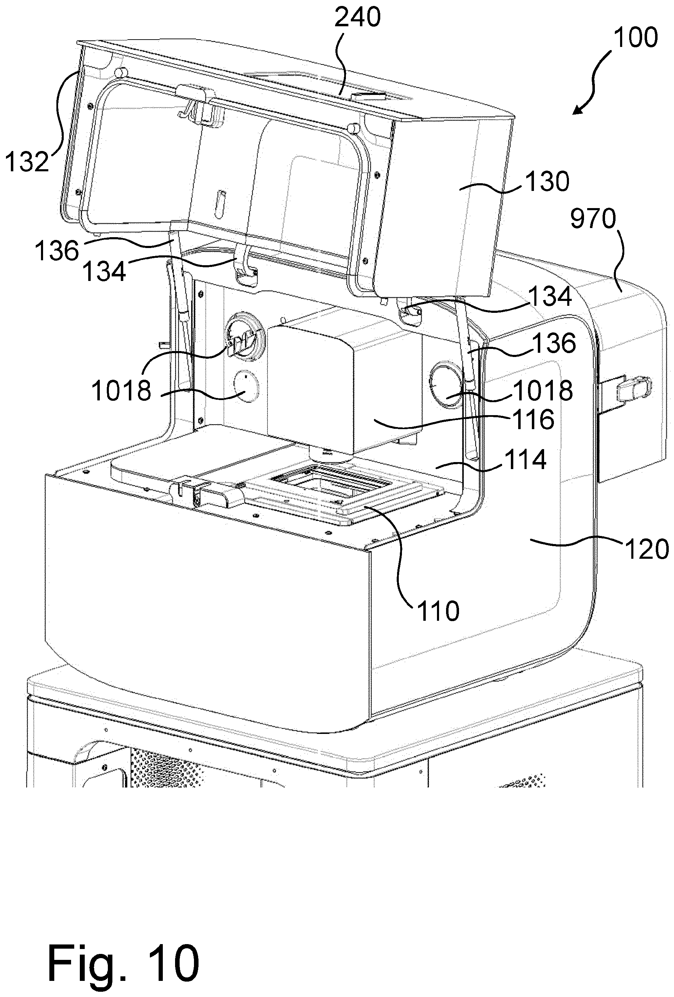

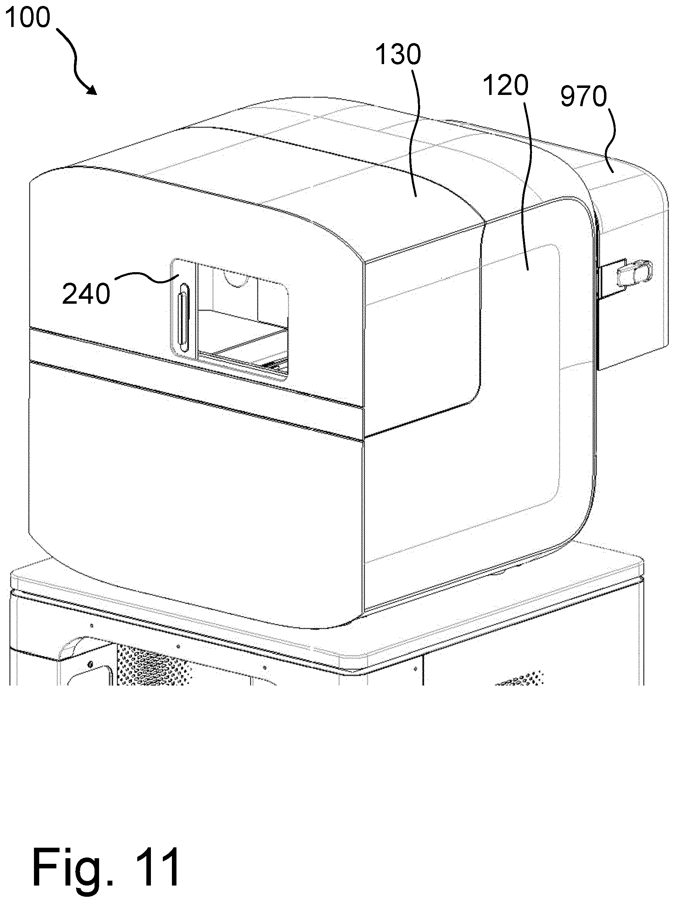

As can be seen from Figure 1, a closed sample chamber 114 is formed by closing the first access door 130. The sample chamber 114 comprises the sample 112, the top side of the microscope stage 110 and an illumination unit 116. The closed sample chamber 114 may be incubated such that a controlled incubation atmosphere of predetermined temperature, humidity and CO₂₋concentration is generated. In this embodiment of a transmitted-light microscope 100, the objective lens of the microscope 100 is arranged below the microscope stage 110. The space below the microscope stage 110 including the objective lens and possibly other imaging devices such as a digital camera may also have available a controlled atmosphere where, for example, only the temperature is controlled in order to avoid condensation of water on optical and electrical components of the imaging unit and other possible temperature-induced damages of such optical and electrical components. Regarding further details about incubation of the sample chamber 114, reference is made to the embodiments of Figures 10 and 11 below.

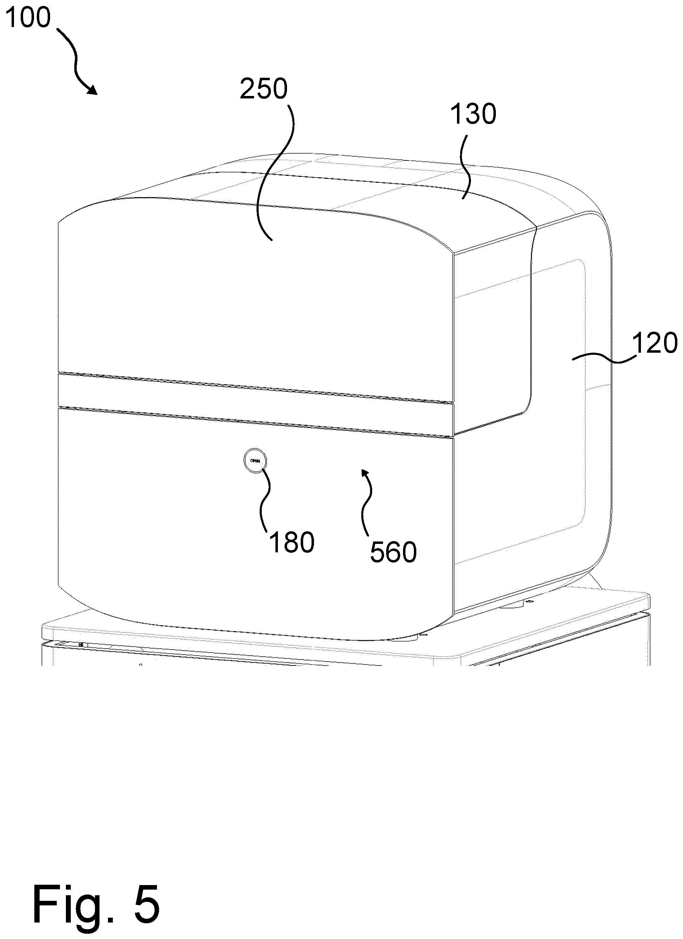

Figures 2 and 3 show the embodiment of Figure 1 with a closed first access door/lid 130. The second access door 240 is a slidable door 240a. A third door 250 is provided, which, in its closed state, covers the second access door 240a when in its closed state, as can be seen from Figures 2 and 5. It can be seen, that the third door 250 does not provide direct access to the microscope stage 110, but the third door 250 can be used as a cover for the second access door, particularly if the second access door 240a is a transparent door, and/or can be used as a work surface 252. To this end, it is preferable if the third door 250 is a hinged door which, in an opened state, is configured as a work surface 252. This configuration can be achieved by hinging down the third door 250 which is stopped in a position as shown in Figure 2 (opening angle of 90°). The essentially horizontal work surface 252 provides a storage area, which can be used for putting down things like reagent containers, laboratory bottles, pipettes, sample containers, etc.

If a transparent second access door 240a is used, a user can examine the interior of the sample chamber 114 while the second access door 240a is closed. Thus, no disturbance of the incubation atmosphere inside the sample chamber 114 occurs. At the same time, light contamination from ambient light is minimized. If a user wants to exchange or manipulate the sample, he/she can open the sliding door 240a. Although this results in a disturbance of the incubation atmosphere, the resulting "leakage" is much smaller compared to an opening of the first access door 130.

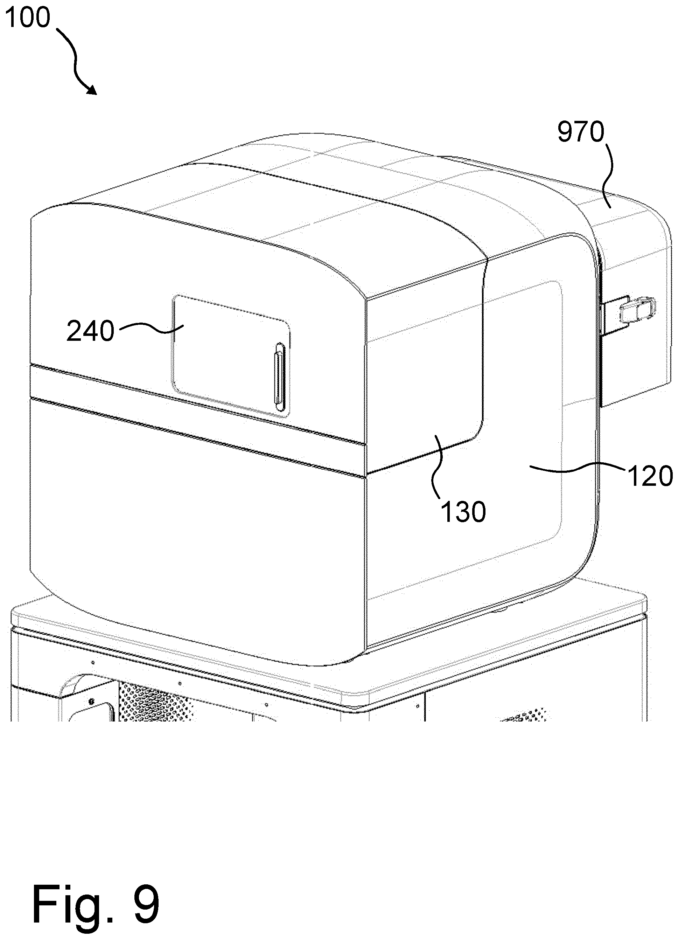

It should be noted that the second access door 240 needs not necessarily to be a sliding door but could also be another door like a hinged door. Sliding door 240a needs not to be transparent but may also be opaque in order to keep any ambient light from the sample in the sample chamber 114. In such a case, visual control of the status of the sample inside the sample chamber 114 may be done by camera inspection. In the embodiment shown in Figures 1 to 3, only in a limited number of cases an opening of the first access door/lid 130 is necessary. Such cases are, for example, setting up an accessory device that should be placed next to the microscope stage, adjusting the illumination unit 116 or setting up an incubation module 970 (see Figure 9).

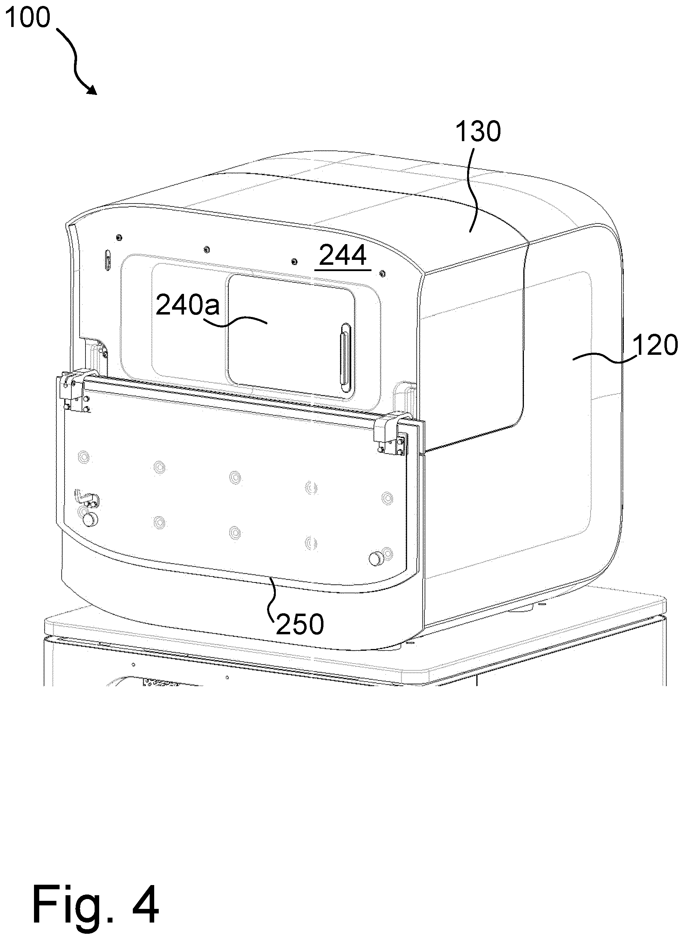

Figure 4 shows another embodiment which is similar to that of Figure 2 with the only difference that the second access door 250 can be hinged down by 180° instead of 90°. Although this yields no table configuration, the access benefits as discussed above are still given. This embodiment creates free space in front of the second access door 240a.

Figure 5 schematically shows an embodiment where preferably all doors of the microscope 100 are closed. The embodiment shown in Figure 5 can e.g. be achieved by closing the first access door 130 of the embodiment of Figure 1 or by closing the third door 250 of the embodiments of Figures 2, 3 or 4. As can be seen from Figure 5, the third door 250, in its closed state, is embodied as at least a part, here the upper part, of a front cover 560 of the microscope housing 120. As can be seen from Figures 2 to 4, the second access door 240a may be formed in a front panel 244 of the microscope housing 120. Thus, the second access door serves for opening and closing the corresponding opening in the microscope housing.

The third door 250 serves for covering the front panel 244 including the second access door 240a. In the embodiment of Figure 5, the third door 250, in its closed state, forms the upper part of the front cover 560 which may be made of a coloured, transparent or semitransparent glass. The microscope 100, in its closed state, forms a closed entity and has the shape of a block.

The (push) button 180 shown in Figures 1 to 5 serves the purpose of unlocking the first access door/lid 130 which can then be opened by a user. It is reasonable to have an interrelated locking mechanism such that the first access door 130 can only be opened if the third door 250 is closed. It may further be reasonable if the third door 250 can only be closed if the second access door 240a is closed, especially in case of an incubated sample chamber.

Further, other interrelated locking mechanisms of the three doors may be provided.

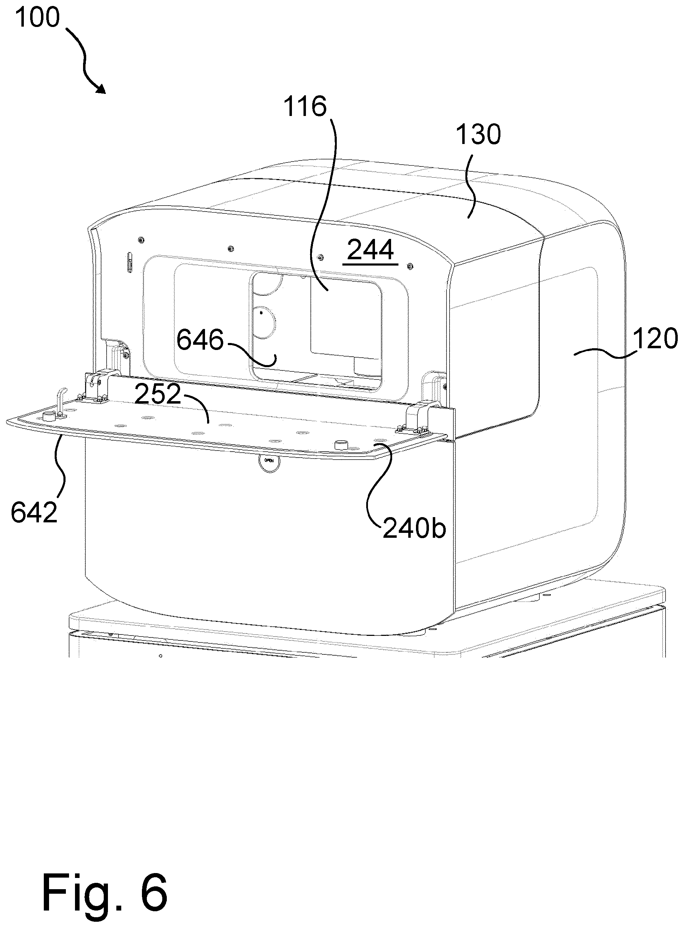

Figure 6 schematically shows another embodiment where the first access door 130 is closed while the second access door 240 is opened. In this embodiment, the second access door 240 is a hinged door 240b. By hinging the second access door 240b down, direct access to the microscope stage 110 becomes possible. The second access door 240b is configured by a second housing part 642 serving the purpose of closing the opening 646 in the front panel 244. As the size of the opening 646 is smaller than the size of the second access door 240b, also a smaller second access door 240b may be used in this embodiment. In the embodiment shown in Figure 6, the front panel 244 together with the closed second access door 240b form parts of the microscope housing 120. Further, in this embodiment, the second access door 240b corresponds to the third door of the embodiments of Figures 1 to 5, the sliding door 240a being left off. Again, a work surface 252 is formed by hinging the second access door 240b down by an opening angle of 90°. Regarding any other features and advantages, especially in regard to the first access door 130, reference is made to the previously discussed embodiments.

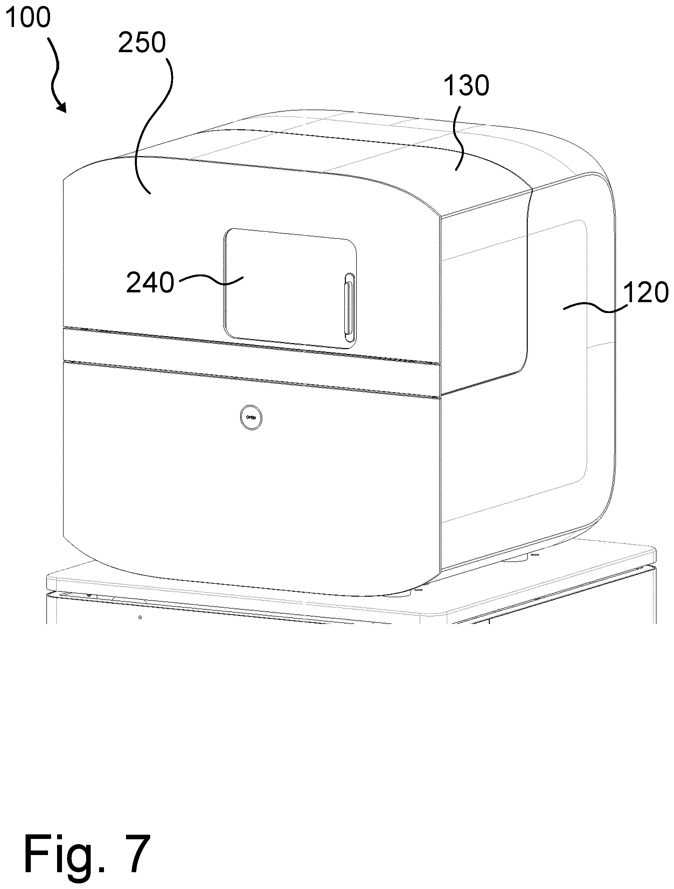

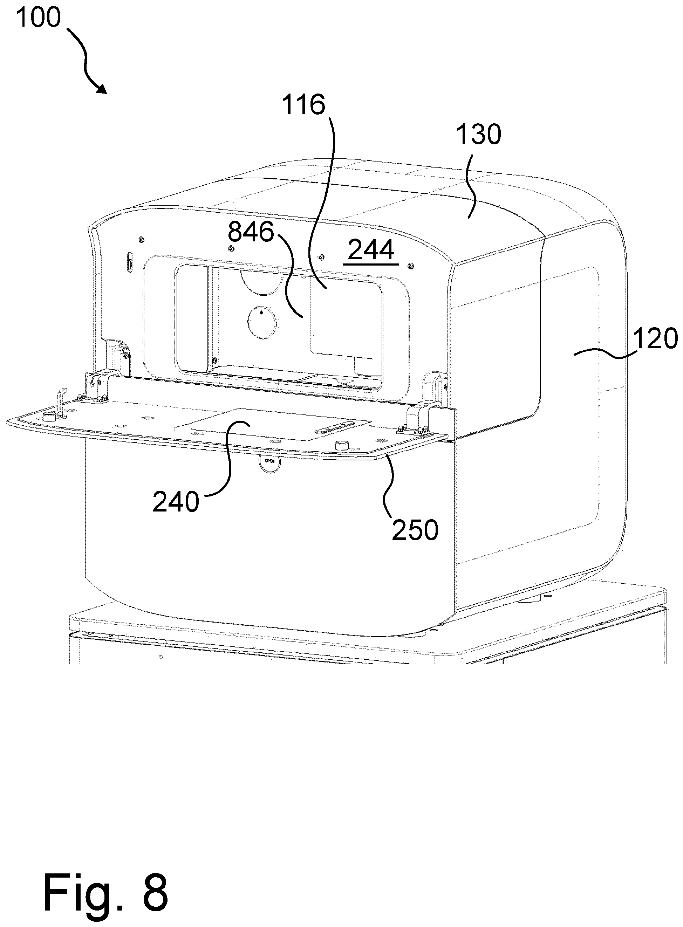

Figure 7 schematically shows yet another embodiment of a microscope, which is not covered by the present claims, where preferably all the doors of the microscope 100 are closed. Figure 8 schematically shows the embodiment of Figure 7 in an opened state where the third door 250 is opened. In this embodiment, which is not covered by the present claims, the microscope 100 comprises a third door 250 which includes the second access door 240. This configuration corresponds to a "door-in-door" configuration in respect of the second access door and the third door, which third door also has the function of an access door. By opening the second access door 240, which is configured as a sliding door, a corresponding opening is uncovered, which provides direct access to the microscope stage 110. In addition, opening the third door 250 uncovers a corresponding opening, which - as can be seen from Figure 8 - exceeds the size of the opening uncovered by the second access door 240. In this embodiment, the opening uncovered by the third door 250 is included in a front panel 244 of the microscope housing 120, however, it would also be possible to enlarge this opening by reducing or even omitting the front panel 244. On the other hand, the size of the third door 250 may be adapted to the size of the opening 846. In the embodiment shown in Figure 8, the third door 250 corresponds to the third door shown in the embodiments of Figures 1 to 3, the third door 250, however, including the second access door 240 as can be seen in Figures 7 and 8, which is not covered by the present claims.

In this embodiment, three different ways of access to the microscope stage 110 are provided to a user. By opening the first access door 130, a wide spherical angle of access to the microscope stage 110 is provided to a user, by opening the third door 250 uncovering the opening 846, a smaller spherical angle of access to the microscope stage 110 is provided, and by opening the second access door 240 an again smaller spherical angle of access is provided. Thus, depending on a user's intention, the user can decide among three different ways of accessibility while, at the same time, trying to keep the level of disturbance of the incubation atmosphere as low as possible.

As can be seen from Figure 7, which is not covered by the present claims, as the second access door 240 is part of the third door 250, and the third door 250 is part of the first access door 130, this configuration forms a "door-in-door" configuration in regard to the first, second and third doors. The third (access) door 250 is coupled to the first access door 130 by a hinge mechanism as already explained in connection with previous embodiments.

Figures 9 to 11 schematically show an embodiment which is similar to the embodiment of Figures 7 and 8 with the main difference that an incubation module 970 is adapted at the backside of the microscope 100. Figure 9 shows this embodiment with closed doors 240 and 130, while Figure 10 shows this embodiment with an opened first access door/lid 130, and Figure 11 shows this embodiment with a closed first access door 130 but with an opened second access door 240. Regarding the features and advantages of the microscope 100 according to this embodiment, reference is made to the discussion of the previous embodiments in order to avoid unnecessary repetitions.

As can be seen from Figure 10, the sample chamber 114 comprises the upper side of the microscope stage 110 and the illumination unit 116. No sample 112 has yet been placed in the microscope stage 110. As previously discussed, the microscope objective (not shown) and further components for imaging a sample, such as a digital camera, are arranged in the lower part of the microscope 100 beneath the microscope stage 110. On the backside of the sample chamber 114 three inlets/outlets 1018 are provided, two of them being closed and one of them being opened and functioning. The three inlets/outlets 1018 inter alia serve the purpose of conducting an incubation atmosphere generated by the incubation module 970 into the interior of the sample chamber 114 and/or out of the sample chamber 114. Further, sensors, tubes for pumps, etc. may be led through one or more of these inlets/outlets 1018. In the embodiment shown in Figure 10, one of these inlets/outlets 1018 is provided with a split tube, one half of this tube being configured for conducting incubation atmosphere into the sample chamber 114, while the other part of the tube being configured for exhausting incubation atmosphere out of the sample chamber 114. In operation, a sample 112 is placed on the microscope stage 110, preferably all of the doors of the microscope 100 are closed, and the incubation module 970 is operated in order to generate an incubation atmosphere of predetermined and controlled temperature, humidity and CO₂₋concentration within the sample chamber 114.

Again, depending on a user's intention, a user may only open the second access door 240 as shown in Figure 11, for instance, in order to exchange the sample 112 or manipulate the sample 112, generating only a small "leakage" with only a small degree of air exchange between the inside and the outside of the sample chamber 114. In contrast, the user may decide to open the first access door/lid 130 for the largest possible access, for instance, for setting up the incubation module or the illumination unit. In this case, there is a high exchange of air between the inside and the outside of the sample chamber, which will most likely destroy an incubation atmosphere if already existing. On the other hand, the user might steer a middle course by opening the third door 250 of the embodiment shown in Figure 8 if this option is implemented in the embodiment of Figures 9 to 11.

### Reference signs

- 100: microscope
- 110: microscope stage
- 112: sample
- 114: sample chamber
- 116: illumination unit
- 120: microscope housing
- 130: first access door
- 132: first housing part
- 134: hinge
- 136: gas spring
- 142: second housing part
- 180: button

- 240: second access door
- 240a: second sliding access door
- 240b: second hinged access door
- 244: front panel
- 250: third door
- 252: work surface

- 560: front cover
- 646: opening
- 642: second housing part
- 846: opening
- 970: incubation module
- 1018: inlet/outlet

## Claims

1. A microscope (100) for examining a sample (112), the microscope (100) comprising a microscope stage (110) configured to receive the sample (112) to be examined and further comprising a microscope housing (120) adapted to enclose the microscope stage (110),
wherein the microscope housing (120) comprises a first access door (130) and a second access door (240, 240a, 240b), whereby both, the first access door (130) and the second access door (240, 240a, 240b), upon opening, are configured to provide different ways of access to the microscope stage (110),
**characterized in that** the second access door (240, 240a, 240b) forms a part of the first access door (130), and
wherein the microscope (100) comprises a third door (250) which, in its closed state, covers the second access door (240a) when in its closed state.

2. The microscope (100) according to claim 1, wherein the opening provided by opening the first access door (130) is larger than the opening provided by opening the second access door (240, 240a, 240b).

3. The microscope (100) according to claim 1 or 2, wherein the first access door (130) is configured by a first housing part (132) of the microscope housing (120) and/or the second access door (240, 240a) is configured by a second housing part (142) of the microscope housing (120).

4. The microscope (100) according to claim 3, wherein the first housing part (132) is larger than the second housing part (142).

5. The microscope (100) according to any one of the preceding claims,
wherein the second access door is a hinged door (240 b) or a sliding door (240, 240a).

6. The microscope (100) according to any one of the preceding claims, wherein the third door (250), in its closed state, is embodied as at least a part of a front cover (560) of the microscope housing (120).

7. The microscope (100) according to any one of the preceding claims, wherein the second access door (240a) is formed in a front panel (244) of the microscope housing (120).

8. The microscope (100) according to any one of the preceding claims, wherein the first access door (130) comprises a hinge (134) to form a hinged lid of the microscope housing (120).

9. The microscope (100) according to claim 3 or any one of the preceding claims, which refers back to claim 3, wherein the first housing part (132) of the microscope housing (120) includes the second housing part (142) of the microscope housing (120).

10. The microscope (100) according to any one of the preceding claims, wherein the third door (250) is coupled to the first access door (130).

11. The microscope (100) according to any one of the preceding claims, which refers back to claim 5, wherein the second access door is a hinged door which, in an opened state, is configured as a work surface (252) and, in a closed state, forms at least a part of the microscope housing (120).

12. The microscope (100) according to any one of the claims 1 to 10, which refers back to claim 6 or 8, wherein the third door (250) is a hinged door which, in an opened state, is configured as a work surface (252) and, in a closed state, forms at least a part of the microscope housing (120).

13. The microscope (100) according to any one of the preceding claims, wherein the microscope housing (110) comprises a sample chamber (114), which sample chamber (114) is bounded at least by the upper side of the microscope stage (110) configured to receive the sample (112), and is further bounded at least by the first access door (130) and by the second access door (240, 240a, 240b), when each of them is in the closed state, and particularly, when referring back to claim 7, wherein the sample chamber (114) is bounded at least by the third door (250) in its closed state.

14. The microscope (100) according to claim 13, wherein the sample chamber (114) comprises and/or is configured to be connected to an incubation module (970) configured to incubate the sample chamber (114).

15. The microscope (100) according to claim 13 or 14, wherein the sample chamber (114) comprises an illumination unit (116) adapted to generate an illumination beam configured to illuminate the sample (112) when received in the microscope stage (110).

## Patentansprüche

1. Mikroskop (100) zum Untersuchen einer Probe (112), wobei das Mikroskop (100) einen Mikroskoptisch (110) umfasst, der zum Aufnehmen der zu untersuchenden Probe (112) konfiguriert ist, und weiter ein Mikroskopgehäuse (120) umfasst, das zum Umschließen des Mikroskoptisches (110) ausgebildet ist,
wobei das Mikroskopgehäuse (120) eine erste Zugangstür (130) und eine zweite Zugangstür (240, 240a, 240b) umfasst, wobei sowohl die erste Zugangstür (130) als auch die zweite Zugangstür (240, 240a, 240b) beim Öffnen so konfiguriert sind, dass sie unterschiedliche Zugangswege zum Mikroskoptisch (110) bieten,
**dadurch gekennzeichnet, dass** die zweite Zugangstür (240, 240a, 240b) einen Teil der ersten Zugangstür (130) bildet, und
wobei das Mikroskop (100) eine dritte Tür (250) umfasst, die in ihrem geschlossenen Zustand die zweite Zugangstür (240a) in ihrem geschlossenen Zustand abdeckt.

2. Mikroskop (100) nach Anspruch 1, wobei die durch das Öffnen der ersten Zugangstür (130) bereitgestellte Öffnung größer ist als die durch das Öffnen der zweiten Zugangstür (240, 240a, 240b) bereitgestellte Öffnung.

3. Mikroskop (100) nach Anspruch 1 oder 2, wobei die erste Zugangstür (130) durch einen ersten Gehäuseteil (132) des Mikroskopgehäuses (120) und/oder die zweite Zugangstür (240, 240a) durch einen zweiten Gehäuseteil (142) des Mikroskopgehäuses (120) gebildet ist.

4. Mikroskop (100) nach Anspruch 3, wobei das erste Gehäuseteil (132) größer ist als das zweite Gehäuseteil (142).

5. Mikroskop (100) nach einem der vorstehenden Ansprüche, wobei die zweite Zugangstür eine schwenkbare Tür (240 b) oder eine Schiebetür (240, 240a) ist.

6. Mikroskop (100) nach einem der vorstehenden Ansprüche, wobei die dritte Tür (250) in ihrem geschlossenen Zustand als mindestens ein Teil einer Frontabdeckung (560) des Mikroskopgehäuses (120) ausgebildet ist.

7. Mikroskop (100) nach einem der vorstehenden Ansprüche, wobei die zweite Zugangstür (240a) in einer Frontplatte (244) des Mikroskopgehäuses (120) ausgebildet ist.

8. Mikroskop (100) nach einem der vorstehenden Ansprüche, wobei die erste Zugangstür (130) ein Scharnier (134) umfasst, um einen schwenkbaren Deckel des Mikroskopgehäuses (120) zu bilden.

9. Mikroskop (100) nach Anspruch 3 oder einem der vorstehenden Ansprüche, der auf Anspruch 3 zurückverweist, wobei der erste Gehäuseteil (132) des Mikroskopgehäuses (120) den zweiten Gehäuseteil (142) des Mikroskopgehäuses (120) einschließt.

10. Mikroskop (100) nach einem der vorstehenden Ansprüche, wobei die dritte Tür (250) mit der ersten Zugangstür (130) gekoppelt ist.

11. Mikroskop (100) nach einem der vorstehenden Ansprüche, der auf Anspruch 5 zurückverweist, wobei die zweite Zugangstür eine schwenkbare Tür ist, die im geöffneten Zustand als Arbeitsfläche (252) konfiguriert ist und im geschlossenen Zustand mindestens einen Teil des Mikroskopgehäuses (120) bildet.

12. Mikroskop (100) nach einem der Ansprüche 1 bis 10, der auf Anspruch 6 oder 8 zurückverweist, wobei die dritte Tür (250) eine schwenkbare Tür ist, die in einem geöffneten Zustand als Arbeitsfläche (252) konfiguriert ist und in einem geschlossenen Zustand mindestens einen Teil des Mikroskopgehäuses (120) bildet.

13. Mikroskop (100) nach einem der vorstehenden Ansprüche, wobei das Mikroskopgehäuse (110) eine Probenkammer (114) umfasst, die Probenkammer (114) mindestens durch die Oberseite des Mikroskoptisches (110), der zum Aufnehmen der Probe (112) konfiguriert ist, begrenzt ist, und weiter mindestens durch die erste Zugangstür (130) und durch die zweite Zugangstür (240, 240a, 240b) begrenzt ist, wenn sich jede von ihnen im geschlossenen Zustand befindet, und insbesondere, wenn auf Anspruch 7 zurückverwiesen wird, wobei die Probenkammer (114) zumindest durch die dritte Tür (250) in ihrem geschlossenen Zustand begrenzt ist.

14. Mikroskop (100) nach Anspruch 13, wobei die Probenkammer (114) ein Inkubationsmodul (970) umfasst und/oder mit einem Inkubationsmodul (970) verbindbar konfiguriert ist, das zum Inkubieren der Probenkammer (114) konfiguriert ist.

15. Mikroskop (100) nach Anspruch 13 oder 14, wobei die Probenkammer (114) eine Beleuchtungseinheit (116) umfasst, die dazu ausgebildet ist, einen Beleuchtungsstrahl zu erzeugen, der so konfiguriert ist, dass er die Probe (112) beleuchtet, wenn diese auf dem Mikroskoptisch (110) aufgenommen wird.

## Revendications

1. Microscope (100) permettant d'examiner un échantillon (112), le microscope (100) comprenant une platine de microscope (110) configurée pour recevoir l'échantillon (112) devant être examiné et comprenant en outre un boîtier de microscope (120) adapté pour renfermer la platine de microscope (110),
dans lequel le boîtier de microscope (120) comprend une première porte d'accès (130) et une deuxième porte d'accès (240, 240a, 240b), ce par quoi, à la fois la première porte d'accès (130) et la deuxième porte d'accès (240, 240a, 240b), lors d'une ouverture, sont configurées pour fournir différentes voies d'accès à la platine de microscope (110),
**caractérisé en ce que** la deuxième porte d'accès (240, 240a, 240b) forme une partie de la première porte d'accès (130), et
dans lequel le microscope (100) comprend une troisième porte (250) qui, dans son état fermé, couvre la deuxième porte d'accès (240a) lorsqu'elle est dans son état fermé.

2. Microscope (100) selon la revendication 1, dans lequel l'ouverture fournie en ouvrant la première porte d'accès (130) est plus grande que l'ouverture fournie en ouvrant la deuxième porte d'accès (240, 240a, 240b).

3. Microscope (100) selon la revendication 1 ou la revendication 2, dans lequel la première porte d'accès (130) est configurée par une première partie de boîtier (132) du boîtier de microscope (120) et/ou la deuxième porte d'accès (240, 240a) est configurée par une seconde partie de boîtier (142) du boîtier de microscope (120).

4. Microscope (100) selon la revendication 3, dans lequel la première partie de boîtier (132) est plus grande que la seconde partie de boîtier (142).

5. Microscope (100) selon l'une quelconque des revendications précédentes, dans lequel la deuxième porte d'accès est une porte articulée (240b) ou une porte coulissante (240, 240a).

6. Microscope (100) selon l'une quelconque des revendications précédentes, dans lequel la troisième porte (250), dans son état fermé, est conçue en tant qu'au moins une partie d'un capot frontal (560) du boîtier de microscope (120).

7. Microscope (100) selon l'une quelconque des revendications précédentes, dans lequel la deuxième porte d'accès (240a) est formée dans un panneau frontal (244) du boîtier de microscope (120).

8. Microscope (100) selon l'une quelconque des revendications précédentes, dans lequel la première porte d'accès (130) comprend une charnière (134) pour former un couvercle articulé du boîtier de microscope (120).

9. Microscope (100) selon la revendication 3 ou l'une quelconque des revendications précédentes, qui fait référence à la revendication 3, dans lequel la première partie de boîtier (132) du boîtier de microscope (120) inclut la seconde partie de boîtier (142) du boîtier de microscope (120).

10. Microscope (100) selon l'une quelconque des revendications précédentes, dans lequel la troisième porte (250) est couplée à la première porte d'accès (130).

11. Microscope (100) selon l'une quelconque des revendications précédentes, qui fait référence à la revendication 5, dans lequel la deuxième porte d'accès est une porte articulée qui, dans un état ouvert, est configurée en tant qu'une surface de travail (252) et, dans un état fermé, forme au moins une partie du boîtier de microscope (120).

12. Microscope (100) selon l'une quelconque des revendications 1 à 10, qui fait référence à la revendication 6 ou la revendication 8,
dans lequel la troisième porte (250) est une porte articulée qui, dans un état ouvert, est configurée en tant qu'une surface de travail (252) et, dans un état fermé, forme au moins une partie du boîtier de microscope (120).

13. Microscope (100) selon l'une quelconque des revendications précédentes, dans lequel le boîtier de microscope (110) comprend une chambre d'échantillon (114), laquelle chambre d'échantillon (114) est délimitée au moins par le côté supérieur de la platine de microscope (110) configurée pour recevoir l'échantillon (112), et est en outre délimitée au moins par la première porte d'accès (130) et par la deuxième porte d'accès (240, 240a, 240b), lorsque chacune d'elles est dans l'état fermé, et en particulier, lorsqu'elle fait référence à la revendication 7, dans lequel la chambre d'échantillon (114) est délimitée au moins par la troisième porte (250) dans son état fermé.

14. Microscope (100) selon la revendication 13, dans lequel la chambre d'échantillon (114) comprend et/ou est configurée pour être reliée à un module d'incubation (970) configuré pour incuber la chambre d'échantillon (114).

15. Microscope (100) selon la revendication 13 ou la revendication 14, dans lequel la chambre d'échantillon (114) comprend une unité d'éclairage (116) adaptée pour générer un faisceau d'éclairage configuré pour éclairer l'échantillon (112) lorsqu'il est reçu dans la platine de microscope (110).
